# EUROPEAN PATENT SPECIFICATION

(11) **EP 2 689 723 B1**
(45) Date of publication and mention of the grant of the patent: **05.09.2018**
(21) Application number: 13177731.0
(22) Date of filing: 24.07.2013
(51) Int. Cl.: A61B 5/107, A61B 90/00, G01B 11/02, G01B 11/25

(54) **Telecentric scale projection system for real-time in-situ surgical metrology**
Telezentrisches Skalaprojektionssystem für die chirurgische in situ Echtzeitmetrologie
Système de projection d'échelle télécentrique pour métrologie chirurgicale in situ en temps réel

(30) Priority: 25.07.2012 US 201261675397 P; 27.06.2013 US 201313928667
(43) Date of publication of application: 29.01.2014
(73) Proprietor: Covidien LP, Mansfield, MA 02048 (US)
(72) Inventor: Pinto, Candido Dionisio, Pacifica, CA 94044 (US); Durvasula, Ravi, Cheshire, CT 06410 (US); Power, James, Houston, TX 77043 (US); Ma, Yong, Cheshire, CT 06410 (US); Pandey, Ashwini, Wallingford, CT 06492 (US); Thomas, Jonathan, New Haven, CT 06511 (US)
(74) Representative: Maschio, Antonio

(56) References cited:
- DE-A1-102010 025 752
- US-A- 4 660 982
- US-A1- 2005 237 423
- US-A1- 2008 068 197

## Description

### BACKGROUND

### Technical Field

The present disclosure relates to a system for measuring a dimension of a desired portion of a surgical site. More particularly, the present disclosure relates to a system for projecting a pattern of a known size onto a desired portion of a surgical site for measuring the desired portion. The pattern may be used to select a suitably sized implant and show desired or optimal fixation points for an implant.

### Background of the Related Art

Minimally invasive surgery, e.g., laparoscopic, endoscopic, and thoroscopic surgery, has many advantages over traditional open surgeries. In particular, minimally invasive surgery eliminates the need for a large incision, thereby reducing discomfort, recovery time, and many of the deleterious side effects associated with traditional open surgery.

The minimally invasive surgeries are performed through small openings in a patient's skin. These openings may be incisions in the skin or may be naturally occurring body orifices (e.g., mouth, anus, or vagina). In general, an insufflation fluid is used to enlarge the area surrounding the target surgical site to create a larger, more accessible work area.

In many surgical situations, having real-time metrology tools providing dimensional measurements would be helpful for surgeons. This is especially the case in minimally invasive surgery where access to the surgical site is limited. The tools can either be stand alone tools or be integrated with surgical instruments. While the size of the metrology tool in most open surgical applications is not as critical, for minimally invasive procedures, it would be helpful to have as small of a form factor as possible.

Both due to accuracy considerations and due to the complex topographies of the surgical site and the need to keep the site as sterile as possible, it would be ideal for the metrology tools to operate in a non-contact fashion.

Surgical implants are often available in various sizes and configurations and metrology tools may be used to select appropriate or optimal implants.

A metrology system including a projection lens system for an endoscope is known from DE 102010025752.

### SUMMARY

The invention provides a metrology system according to claim 1. The current disclosure describes several embodiments of endoscopic metrology tools which can be realized in a small form factor and employ non-contact methods for dimensional measurements. These embodiments primarily exploit optical and/or acoustical methods.

An aspect of the present disclosure provides a system for measuring a dimension of a desired portion of a surgical site including a projector assembly which includes a light source for projecting light through a telecentric lens and into the surgical site, and a mask operably coupled to the projector assembly. The light projected from the light source projects through the mask. The projected light through the mask is a collimated pattern which does not change in size as a function of a distance to a projected plane, i.e., the desired portion of the surgical site. The projected light may include multiple wavelengths of light for measurement of different features of tissue within the surgical site. The mask may include a scale which projects onto the desired portion of the surgical site. The mask may have concentric rings each of which represents a radius of a given dimension. The light source may include at least one lighting element. The light source may further include a diffuser for diffusing the light produced by the at least one lighting element. The mask may include a collimated pattern for projecting the collimated pattern onto the desired portion of the surgical site. The telecentric lens and/or the mask may be formed of a flexible material. The system may further include a polymetric scale positioned external to the surgical site for projecting the scale through the tissue for viewing within the surgical site.

Additionally or alternatively, another aspect of the present disclosure which does not describe part of the claimed invention provides an imaging unit for capturing an image of the projected light in the surgical site. The imaging unit may be a CMOS camera and/or a raster scanning device. Additionally or alternatively, a microprocessor may be coupled to the imaging unit, and the microprocessor may perform parallax corrections of the captured image. The microprocessor may be capable of calculating measurement dimensions of the desired portion of the surgical site. A display may be coupled to the microprocessor and the dimensions calculated by the microprocessor may be displayed on the display. Additionally or alternatively, the system may further include a sensor for performing triangulation or distance sensing. Additionally or alternatively, an interferometer may be coupled to the sensor. The measurements of the desired portion of the surgical site may be transmitted to an implant printing device for creating, for example, a surgical mesh according to the measurements or for marking a mesh with desired points for fixation by tacks, sutures or other mesh.

Another aspect of the present disclosure which does not describe part of the claimed invention provides a method for measuring a desired portion of a surgical site including projecting light from a projector assembly into a surgical site and analyzing the projected light. The projector assembly may include a light source for projecting the light through a telecentric lens, and a mask operably coupled to the projector assembly. The light projected from the light source projects through the mask. The projected light through the mask may be a collimated pattern which does not significantly change in size as a function of a distance to a projected plane, i.e., the desired portion. The projected light may include multiple wavelengths of light, and the analyzing step may include measuring different features of tissue within the surgical site by comparing the different wavelengths of light. Additionally or alternatively, the mask may have a scale and the scale is projected onto the desired portion of the surgical site, and the analyzing step includes measuring the desired portion of the surgical site by comparing the desired portion with the projected scale. Additionally or alternatively, the mask may have concentric rings, each ring representing a radius of a given dimension, and the concentric rings are projected on a desired portion of the surgical site, and the analyzing step includes measuring the desired portion of the surgical site by comparing the desired portion with the concentric rings. The light source may have at least one lighting element and/or may include a diffuser for diffusing the light produced by the at least one lighting element. Additionally or alternatively, the mask may include a collimated pattern for projecting the collimated pattern onto the desired portion of the surgical site, and the analyzing step may include measuring the desired portion of the surgical site by comparing the desired portion with the collimated pattern. The pattern may correspond to a known or a series of known implant sizes corresponding to available mesh sizes. The telecentric lens and/or the mask may be formed of a flexible material. Additionally or alternatively, the method may further including positioning a polymetric scale external to the surgical site and projecting a scale through tissue for viewing within the surgical site. Thus, for example, the fixation points for a mesh may be projected from inside the abdomen through tissue to allow suturing or fixation from outside the abdomen.

Additionally or alternatively, another aspect of the present disclosure which does not describe part of the claimed invention provides the method described above further including capturing an image of the projected light in the surgical site via an imaging unit. The imaging unit may be a CMOS camera and/or a raster scanning device. The method may further include performing parallax corrections of the captured image via a microprocessor operatively coupled to the imaging unit. The method may further include calculating measurement dimensions of the desired portion of the surgical site. The method may further include displaying the calculated measurement dimensions on a display operatively coupled to the microprocessor. The method may further include performing triangulation or distance sensing via a sensor. An interferometer may be operatively coupled to the sensor. The method may further include selecting an implant based on the measurement dimensions. Throughout this specification an implant may be a mesh, such as a hernia mesh, a non-woven device, a film, a tissue engineering scaffold and other types of implants. Where mesh is used as an example, other suitable implants may be substituted. Implants may be rapid prototyped using methods such as 3-D printing. For example, the method may further include transmitting the calculated measurement dimensions to a mesh printing device and creating a surgical mesh according to the measurements. The created mesh may include optimal fixation points.

### BRIEF DESCRIPTION OF THE DRAWINGS

The above and other aspects, features, and advantages of the present disclosure will become more apparent in light of the following detailed description when taken in conjunction with the accompanying drawings in which:
FIG. 1 is a side, schematic view of a metrology system according to the principles of the present disclosure;
FIG. 2A is front view of a mask of the metrology system of FIG. 1 in accordance with one embodiment of the present disclosure;
FIG. 2B is front view of a mask of the metrology system of FIG. 1 in accordance with another embodiment of the present disclosure;
FIG. 2C is front view of a mask of the metrology system of FIG. 1 in accordance with another embodiment of the present disclosure;
FIG. 2D is front view of a mask of the metrology system of FIG. 1 in accordance with another embodiment of the present disclosure;
FIG. 3 is a side, schematic view of a metrology system according to an embodiment of the present disclosure;
FIG. 4 is a side, schematic view of a metrology system inserted in a surgical site according to an embodiment of the present disclosure;

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end or portion of the system and/or apparatus which is closer to the user and the term "distal" refers to the end or portion of the system and/or apparatus which is farther away from the user. The term "clinician" or "user" refers to any medical professional (i.e., doctor, surgeon, nurse, or the like) performing a medical procedure involving the use of embodiments described herein.

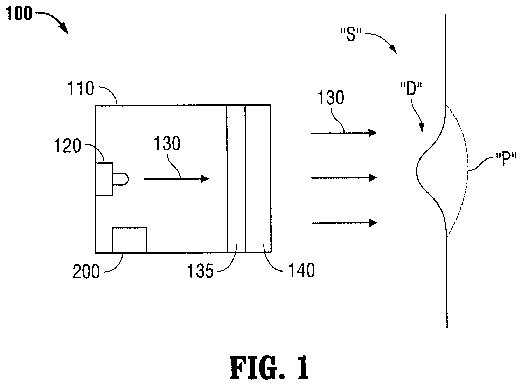

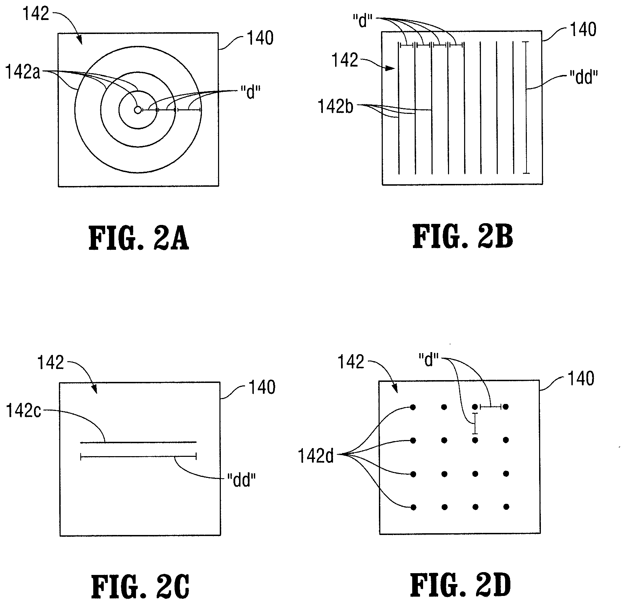

As shown in Fig. 1, metrology system 100 includes a projector assembly 110 which is configured to couple with an endoscopic surgical device (not shown). Projector assembly 110 includes a light source which includes at least one light emitter 120 such as, for example, LED, laser diode or any combination thereof, for emitting light beams 130, a telecentric lens 135, and a mask 140. Although telecentric lens 135 and mask 140 are shown as separate components, it is also envisioned that telecentric lens 135 may include a mask 140 and/or mask 140 may include a telecentric lens 135. Light emitter 120 is disposed on the proximal end of projector assembly and light beams 130 are directed in a forward, i.e., distal, direction. Telecentric lens 135 is disposed distal to light emitter 120 such that light beams 130 pass through telecentric lens 135. Mask 140 is disposed distal to telecentric lens 135 such that light beams 130 also pass through mask 140 and into the surgical site "S." Mask 140 may include certain features or patterns 142 (Figs. 2A-2D) for projecting a light pattern "P" on a desired portion "D" of the surgical site "S." As described above, although mask 140 is shown as disposed distal to telecentric lens 135, telecentric lens 135 may be disposed distal to mask 140. The desired portion "D" of the surgical site "S" may include any feature of the surgical site "S," such as, without limitation, a lesion, herniated defect, or any other anatomical features that may be present within the surgical site "S" and which are desired to be measured by a user.

Telecentric lens 135 and/or mask 140 may be formed of a flexible material to aid in inserting projector assembly 110 into surgical site "S." Suitable lenses may include, for example and without limitation, a foldable imaging lens, a rollable lens, and/or an intra-ocular pseudophakic implant. A telecentric lens 135 may be a compound lens which has an entrance and/or an exit pupil at infinity which decouples the dependency of magnification of an image. This produces a chief ray which is parallel to the optical axis in the space of interest, and a constant magnification in the case of a system which is telecentric in image-space. An entrance pupil at infinity makes the telecentric lens 135 object-space telecentric which causes image magnification to be independent of the object's distance or position in the field of view. An exit pupil at infinity makes the telecentric lens image-space telecentric. Additionally, both an entrance pupil at infinity and an exit pupil at infinity makes the telecentric lens 135 double telecentric.

Continuing with reference to Fig. 1, mask 140 may include a light shaping optical diffuser, a spatial filter, or any other suitable object known in the art capable of scattering and/or spreading light. Additionally or alternatively, light shaping optical diffuser, spatial filter, and/or any other suitable object known in the art may be disposed in projector assembly 110 as a separate component from mask 140 and/or light shaping optical diffuser, spatial filter, and/or any other suitable object known in the art may be incorporated into telecentric lens 135. Additionally or alternatively, light shaping optical diffuser and/or spatial filter may be disposed proximal to mask 140 and/or telecentric lens 135. Each light emitter 120 emits a light beam 130 for projecting a light pattern "P" on a desired portion "D" of a surgical site "S." Light emitter 120 creates light beam 130 and the use of multiple light emitters 120 creates multiple light beams 130. Light beam 130 diffuses, i.e., scatters and/or spreads, upon passing through light shaping optical diffuser, spatial filter, or any other suitable object known in the art, such that light beam 130 may be even distributed through telecentric lens 135 and/or mask 140. With the evenly distributed or scattered light beam 130 passing through telecentric lens 135 and/or mask 140, the light pattern "P," which is created by patterns 142 (Figs. 2A-2D) on mask 140, is not magnified or degraded when projected into the surgical site "S."

Continuing with reference to Fig. 1, adjacent light beams 130 have a fixed distance therebetween. Light beams 130 may be collimated for increased precision of the light pattern "P" which is projected on a desired portion "D" of the surgical site "S," or light beams 130 may be scattered or diffused as described above. Light beam 130 may be any suitable form of light, such as coherent, partially coherent, visible, infrared, or ultraviolet. Light beam 130 may have a wavelength of, for example, 532 nm, to differentiate light beams 130 from a color of any naturally occurring tissue in the human body. Additionally or alternatively, light beams 130 may be multiple wavelengths of light for measurement of different features or for simultaneously outlining margins of a desired portion "D" of the surgical site "S," i.e., diseased tissue.

Light emitters 120 are powered by a power source 200. As shown in Fig. 1, power source 200 may be disposed within projector assembly 110. Additionally or alternatively, power source 200 may be positioned in different locations, such as, for example, within a device (not shown) that projector assembly 110 is coupled to. The power source 200 may be a standard commercial battery pack or any other suitable power source known in the art capable of supplying power to light emitter 120. Additionally or alternatively, light emitter 120 may emit light beams 130 without a power source 200, for example by chemically produced light.

Turning now to Figs. 2A-2D, mask 140 may be semi-transparent and/or may have a substantially opaque mask pattern 142 thereon. Mask patterns 142 may have fiducials or markings of known distances therebetween, and/or may further include a scale to aid in visual measurements. For example, mask pattern 142 may be a series of uniformly spaced concentric circles 142a (Fig. 2A), uniformly spaced lines 142b (Fig. 2B), a single line 142c (Fig. 2C), or uniformly spaced dots 142d (Fig. 2d). Additionally, or alternatively, the actual dimensions of the known distances "d" may also be projected. When the actual dimensions are projected with the light pattern "P," a user, i.e., a surgeon, may directly view the measurement of the desired portion "D" when the pattern "P" is projected directly on the desired portion "D" of the surgical site "S." It is understood that the mask patterns 142 may take on multiple shapes and forms beyond those described in this description and illustrated in the drawings. Additionally or alternatively, although mask 140 is shown to be substantially square in shape, it is envisioned that mask 140 may take the form of any shape.

Turning specifically to Fig. 2A, the front of mask 140 is shown with pattern 142 as uniformly spaced concentric circles 142a. Four uniformly spaced concentric circles 142a are shown, each having a uniform distance "d" between them. Although four uniformly spaced concentric circles 142a are shown in Fig. 2A, mask 140 may include any number of uniformly spaced concentric circles 142a. For example, mask 140 may include only a single circle 142a and may have a known diameter. Although not explicitly shown, the actual measurement of the distance "d" between each of the uniformly spaced concentric circles 142a may also be included in pattern 142 such that when pattern 142 is projected into surgical site "S" the actual distances "d" will also be projected and thus will also be visible by a user for a visible measurement within the surgical site "S."

Turning now specifically to Fig. 2B, the front of mask 140 is shown with pattern 142 as uniformly spaced lines 142b. Eight uniformly spaced lines 142b are shown, each having a uniform distance "d" between them. Although eight uniformly spaced lines 142b are shown in Fig. 2B, mask 140 may include any number of uniformly spaced lines 142b. Additionally, although not explicitly shown, the actual measurement of the distance "d" between each of the uniformly spaced lines 142d may also be included in pattern 142 such that when pattern 142 is projected into surgical site "S" the actual distances "d" will also be projected and thus will also be visible by a user for a visible measurement within the surgical site "S."

Continuing with reference to Fig. 2B, although not explicitly shown, the actual measurement "dd" of the length of each individual uniformly spaced line 142b may also be included in the pattern 142 such that when pattern 142 is projected into surgical site "S" the actual lengths "dd" will also be projected and thus will also be visible by a user for a visible measurement within the surgical site "S." Although uniformly spaced lines 142b are shown as having the same length "dd" as all of the other uniformly spaced lines 142b, each uniformly spaced line 142b may include a different length "dd" from the other uniformly spaced lines 142b.

Continuing with reference to Fig. 2B, uniformly spaced lines 142b are shown as uniformly spaced columns extending vertically i.e. downward/upward. It is also envisioned that uniformly spaced lines 142b may take the form of rows extending horizontally i.e. side to side. Additionally or alternatively, uniformly spaced lines 142b may include both vertically extending lines and horizontally extending lines. When both the horizontal and vertical lines are present, the horizontal lines may extend across one or more of the vertical lines, and vise-versa. Additionally or alternatively, a portion of the mask 140 may include horizontally extending lines while another portion of the mask 140 may include the vertically extending lines. Additionally or alternatively, the vertically extending lines may intersect the horizontally extending lines in a perpendicular manner or they may intersect with each other in a non-perpendicular manner.

Turning now to Fig. 2C, the front of mask 140 is shown with pattern 142 as a single line 142c. The single line has a length "dd," and the actual measurement of length "dd" may also be included in pattern 142 such that when pattern 142 is projected into surgical site "S" the actual length "dd" will also be projected and thus will also be visible to a user for a visible measurement within the surgical site. Additionally or alternatively, a single fiducial and/or scale or a plurality of fiducials and/or scales may be included on the single line 142c with known distances between them for more accurate measurements.

Turning now to Fig. 2D, the front of mask 140 is shown with pattern 142 as uniformly spaced dots 142d. Each uniformly spaced dot 142d has a distance "d" between the adjacent uniformly spaced dots 142d. Although sixteen uniformly spaced dots 142d are shown in Fig. 2D, mask 140 may include any number of uniformly spaced dots 142d. Additionally, although not explicitly shown, the actual measurement of the distance "d" between each of the uniformly spaced lines 142d may also be included in pattern 142 such that when pattern 142 is projected into surgical site "S" the actual distances "d" will also be projected and thus will also be visible by a user for a visible measurement within the surgical site "S."

Any of the patterns 142 described above with respect to Figs. 2A-2D may further include a scale, such as for example fiducials, and the scale may be projected with the pattern 142 on to the desired portion "D" of the surgical site "S." With a scale projected onto the desired portion "D," a user may measure the desired portion "D" by placing, i.e. aiming, the projected pattern "P" onto the desired portion "D" of the surgical site "S."

Additionally or alternatively, a large telecentric laser illuminator may be utilized, at a red or near-infrared wavelength, as the projection through a large, flexible polymetric scale in contact with the external skin of a patient. Such implementation enables a projection of the scale itself, though organic layers and minimal scattering losses to be captured by the surgeon's laparoscopic camera and internal defects may still be measured inside or outside the body cavity before the mesh size is chosen to match it in utility for closure/repair.

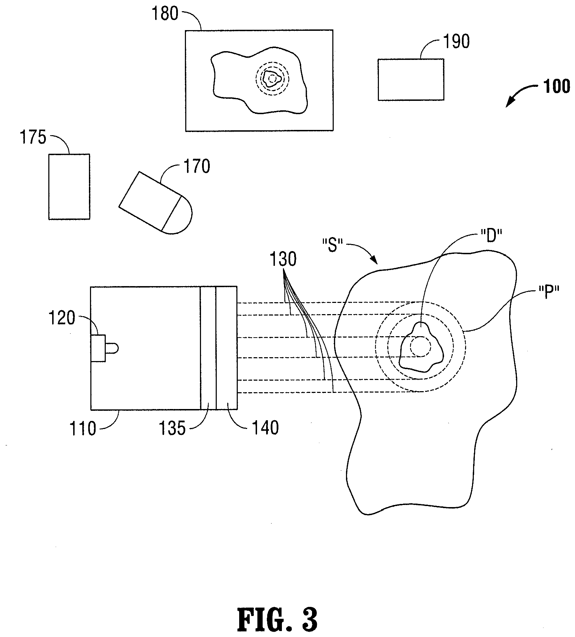

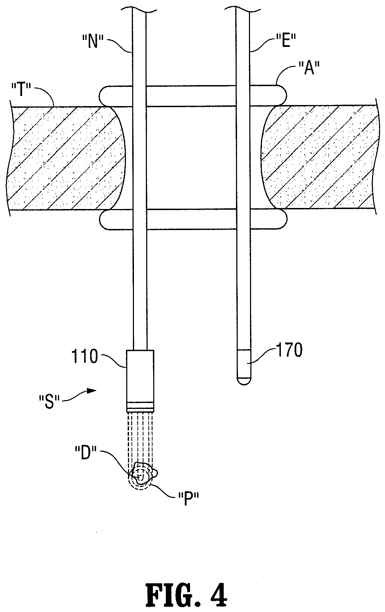

Turning now to Fig. 3, system 100 may further include an imaging unit 170 configured to capture an image, i.e. a pixelized image, or series of images, of the surgical site "S." In particular, imaging unit 170 may capture an image or images of the projected pattern "P" created by projector assembly 110. As shown in Fig. 3, the projected pattern "P" is projected directly onto the desired portion "D" of the surgical site "S." Additionally or alternatively, although not explicitly shown, the projected pattern "P" may be projected adjacent to the desired portion "D" of the surgical site "S." Imaging unit 170 may be a CMOS camera, a raster scanning device, or any other suitable imaging unit known in the art. Imaging unit 170 may be disposed within projector assembly 110, may be operably coupled to projector assembly 110, or may be a separate unit from projector assembly 110. A telecentric lens may also be a part of the imaging unit (170 from Fig. 3 and 4) which captures an image of the projected pattern produced by the projector assembly.

Continuing with reference to Fig. 3, system 100 may further include a microprocessor 175 operably coupled to the imaging unit 170. The imaging unit 170 transmits the captured image, or images, of the surgical site "S" to the microprocessor 175 via a wired connection or wirelessly. Although microprocessor 175 is shown as a separate component from imaging unit 170, it is also envisioned that microprocessor 175 may be the same unit as imaging unit 170 and/or imaging unit 170 may be capable of performing all of the functions of microprocessor 175. Because imaging unit 170 may not be viewing the projected patterns "P" on the surgical site "S" from the same perspective, i.e. the same viewing angle, as the projector assembly 110, microprocessor 175 is configured to perform parallax corrections of the captured image or images transmitted by the imaging unit 170. Additionally, or alternatively, as described above, imaging unit 170 may be configured to perform parallax corrections. Microprocessor 175 may be configured to analyze the image captured by imaging unit 170 and calculate measurement dimensions of the desired portion "D" of the surgical site "S."

Alternatively or additionally, microprocessor 175 may employ triangulation techniques to assess the relative distances between the projector assembly 110 and/or imaging unit 170 and the desired portion "D" of the surgical site "S." Triangulation could be obtained in multiple ways including using a single imaging device 170, multiple imaging devices, or a combination of an imaging device(s) 170 and collimated light sources. Additionally or alternatively, optical and/or acoustical methods can also be employed for range finding, an example of which would be optical or acoustical interferometers (not explicitly shown) and/or sensors (not explicitly shown). For additional accuracy, metrology may be performed from multiple known relative angles. In applications for which triangulation and/or distance sensing is desirable, a fringe-counting heterodyne interferometer may be implemented, with the aide of a LED or laser source, along with a Si or GaAs-based sensor.

Continuing with reference to Fig. 3, system 100 may further include a display 180 operatively coupled to the microprocessor 175. Additionally or alternatively, the display 180 may be operably coupled to the imaging unit 170. It is envisioned that the display 180 may be a graphical user interface and that the display 180 may be integrated with the surgical endoscope of which the projector assembly 110 and/or imaging unit 170 is attached so that the user may view the images and/or calculated measurements produced on the display 180 directly on the instrument, i.e. surgical endoscope. Display 180 displays the image captured by imaging unit 170. Additionally or alternatively, the display 180 may display the measurement dimensions calculated by the microprocessor 175 and/or imaging unit 170.

Continuing with reference to Fig. 3, system 100 may further include a rapid prototyping or printing device 190 operably coupled to the microprocessor 175, imaging device 170, and/or display 180 via a wired connection or wirelessly. Printing device 190 may be, for example and without limitation, a laser cutter or weaving machine, which can produce a customized implant or substrate, however printing device 190 may be any printing device known in the art. Microprocessor 175 transmits the image captured by imaging unit 170 to printing device 190 for printing the image captured onto a substrate and/or creating the substrate. The substrate may be, for example and without limitation, a surgical mesh. The substrate, i.e. surgical mesh, may include porous fabrics made from intertwined filaments, where the printing device 190 is configured to intertwine the filaments. The filaments may be monofilaments or multi-filaments and, in embodiments, a plurality of multi-filaments may be combined to form yarns. The filaments may extend horizontally and vertically in a manner which produces sections where the filaments cross-over one another creating points of common intersection. The substrate, i.e. surgical mesh, may be woven, non-woven, knitted or braided. In some embodiments, the filaments may form two-dimensional or three-dimensional meshes. The printing device 190 prints and or otherwise creates the substrate, i.e. surgical mesh, according to the calculations, measurements, and images captured and created by the imaging unit 170 and/or microprocessor 175. The mesh may be printed with optimal fixation or cardinal points determined by the surgeon or by the computer determined by expert system algorithms. The points may be calculated to estimate the effect of deflation of the abdomen such that the dimished diameter allows the mesh to lie correctly on the tissue rather than, for example,creating folds.

With continued reference to Fig. 3, light emitter 120 emits light beams 130 to create projected pattern "P" on the desired portion "D" of surgical site "S." As mentioned above, the projected pattern "P" may include actual dimensions of the projected shapes. Although Fig. 3 illustrates uniformly spaced concentric circles 142a (Fig. 2A), as described above, the projected pattern "P" may include any of the patterns described in Figs. 2A-2D, or any combinations thereof.

Turning now to Fig. 4, and continuing with reference to Fig. 3, a method of use of metrology system 100 will now be described. As seen in Fig. 4, a desired portion "D" to be measured exists within a surgical site "S" under tissue "T." Projector assembly 110 of metrology system 100 may be attached to a distal end of a surgical instrument "N." Surgical instrument "N" is inserted through a surgical access port "A" positioned in an opening in tissue "T." An endoscope "E" is inserted through surgical access port "A" for viewing surgical site "S." As described above, endoscope "E" may be the imaging unit 170 of system 100. Additionally or alternatively, imaging unit 170 may be operably coupled to endoscope "E" and/or surgical instrument "N."

Continuing with reference to Figs. 3 and 4, and as described above, light emitter 120 of projector assembly 110 emits light beams 130 through telecentric lens 135 and/or mask 140. The pattern 142 of mask 140 determines the corresponding pattern "P" projected into the surgical site "S," specifically onto the desired portion "D" of the surgical site "S." The light beams 130 may include multiple wavelengths of light for measurement of different features or for simultaneously outlining margins of a desired portion "D" of the surgical site "S," i.e., diseased tissue. A user may analyze the desired portion "D," for example, by viewing the different wavelengths projected onto the desired portion "D."

After the projected pattern "P" is projected into the surgical site "S," a user may analyze the projected pattern "P." The user may view the projected pattern "P" on the desired portion "D" within the surgical site "S" on the display 180. In addition to viewing the surgical site "S" on the display 180, the user may also view the calculated measurement dimensions of the desired portion "D," which are calculated by the microprocessor 175, on the display 180. With the projected pattern "P" on the desired portion "D," a user may analyze the actual size of the desired portion "D" in several different ways which are described in further detail below.

In particular, when the mask 140 has concentric rings 142a (Fig. 2A), each ring 142a represents a radius of a given dimension, and the concentric rings 142a are projected into the surgical site "S" as projected pattern "P," the user may analyze and/or measure the desired portion "D" by comparing the desired portion "D" with the concentric rings 142a. Additionally or alternatively, when the mask 140 includes uniformly spaced lines 142b (Fig. 2B), and the uniformly spaced lines 142b are projected into the surgical site "S" as projected pattern "P," the user may analyze and/or measure the desired portion "D" by comparing the desired portion "D" with the uniformly spaced lines 142b. Additionally or alternatively, when the mask 140 includes a single line 142c (Fig. 2c), and the single line 142c is projected into the surgical site "S" as projected pattern "P," the user may analyze and/or measure the desired portion "D" by comparing the desired portion "D" with the single line 142c. Additionally or alternatively, when the mask 140 includes uniformly spaced dots 142d (Fig. 2D), and the uniformly spaced dots 142d are projected into the surgical site "S" as projected pattern "P," the user may analyze and/or measure the desired portion "D" by comparing the desired portion "D" with the uniformly spaced dots 142d. As previously described, the projected pattern "P" may include a scale and/or fiducials to aid in the measurements.

As described above, specific fiducials and/or scales can be projected on the surgical site "S" which can be imaged on to a pixel arrayed sensor of the image where based on prior knowledge of the relative size and shape or location of the fiducials, image processing algorithms establish dimensional features of interest on the target site "S." These features of interest include, for example, optimal fixation points and cardinal points for attaching the implant. For additional accuracy, although not shown, metrology may be performed from multiple known relative angles.

As described above, telecentric lens 135 and/or mask 140 may be formed of a flexible material. In a case where telecentric lens 135 and/or mask 140 are formed of a flexible material a user may reduce the size, for example by rolling or folding, of the telecentric lens 135 and/or mask 140 for insertion into the surgical site "S." Subsequent to being inserted into the surgical site "S," the telecentric lens 135 and/or mask 140 may be brought back to the original shape for projecting light beams 130 into the surgical site "S."

Use of a telecentric lens 135 enables the projection pattern "P" to be telecentric in image space. This features allows, for example, the scale 142 (Figs. 2A-2D) of mask 140 to be directly projected onto any portion of the surgical site "S," i.e. any organ during surgery, creating a direct measurement of any desired portion "D" to the user, independent of any magnification variations in the imaging unit 170 optics used to capture the image of the projected pattern "P."

The projected image, cardinal points and other fiducials may be of sufficient brightness to illuminate through tissue and or the mesh to allow the surgeon to distinguish these features externally through the abdomen or intermediate tissue and fascial layers. Thus, the mesh can be optimally positioned internally, illuminated with the desired pattern and the pattern visualized externally to allow accurate fixation from the outside of the peritoneum to the inside of the peritoneum.

As can be appreciated from the foregoing description and drawings, embodiments of an optical metrology and image correction system according to the present disclosure have been described which yield methods for real-time in-body-cavity metrology employing visible, ultraviolet or near-infrared (IR) radiation, which is either coherent or incoherent, to reduce overall surgery time and the cognitive burden on the surgeon. The embodiments also potentially improve patient outcome with more accurate, smaller (depending on the miniaturization scale) incision procedures, which are less prone to human errors or miscalculations.

Improvements in the surgical procedures originate from both savings in time and from more accurate surgical choices by a given surgeon when attempting to choose measurement-dependent devices for a give in-body task or procedure, such as mesh size during a hernia repair.

## Claims

1. A metrology system for measuring a desired portion (D) of a surgical site (S), comprising:
a telecentric lens (135),
a projector assembly (110) comprising a light source for projecting light through the telecentric lens (135) and into the surgical site (S); and
a mask (140) operably coupled to the projector assembly (110), wherein light projected from the light source projects through the mask (140),
**characterized in that** the mask (140) comprises a pattern (142) for projecting onto the desired portion (D) of the surgical site (S), the telecentric lens (135) configured to project the pattern (142) such that the pattern (142) is not magnified when projected onto the desired portion (D) of the surgical site (S).

2. The system of claim 1, wherein the projected light includes multiple wavelengths of light for measurement of different features of tissue within the surgical site (S).

3. The system of claim 1 or claim 2, wherein the pattern (142) has concentric rings (142a) and each ring (142a) represents a radius of a given dimension for projecting the concentric rings into the surgical site (S).

4. The system of any of the preceding claims, wherein the pattern (142) has a plurality of uniformly spaced lines (142b) for projecting the uniformly spaced lines into the surgical site (S).

5. The system of any of the preceding claims, wherein the pattern (142) has a single line (142c) for projecting the single line into the surgical site (S).

6. The system of any of the preceding claims, wherein the pattern (142) has uniformly spaces dots (142d) for projecting the uniformly spaced dots into the surgical site (S).

7. The system of any of the preceding claims, wherein the pattern (142) includes a scale and the scale is projected onto the desired portion (D) of the surgical site (S).

8. The system of any of the preceding claims, wherein the light source includes at least one light emitter (120).

9. The system of any one of the preceding claims, wherein the light source further includes a diffuser for diffusing the light produced by the at least one light emitter (120).

10. The system of any of the preceding claims, wherein the telecentric lens (135) is formed of a flexible material to aid in inserting into surgical site (S).

11. The system of any of the preceding claims, wherein the mask (140) is formed of a flexible material to aid in inserting into surgical site (S).

12. The system of any of the preceding claims, further having an imaging unit (170) configured to capture an image of the projected light in the surgical site (S).

13. The system of claim 12, wherein the imaging unit (170) is a CMOS camera.

14. The system of claim 12 or claim 13, wherein the imaging unit (170) is a raster scanning device.

15. The system of any of claims 12 to 14, further including a microprocessor (175) operatively coupled to the imaging unit (170), the microprocessor (175) configured to perform parallax corrections of the captured image.

16. The system of claim 15, further including a display (180) operatively coupled to the microprocessor (175), the microprocessor (175) configured to calculate measurement dimensions of the desired portion (D) of the surgical site (S) and transmit the measurement dimensions to the display (180).

17. The system of any of the preceding claims, further having a sensor configured to perform triangulation or distance sensing.

## Patentansprüche

1. Metrologiesystem zum Messen eines gewünschten Abschnitts (D) eines Operationssitus (S), umfassend:
ein telezentrisches Objektiv (135),
eine Projektoranordnung (110), umfassend eine Lichtquelle zum Projizieren von Licht durch das telezentrische Objektiv (135) und in den Operationssitus (S); und
eine Maske (140), die wirksam mit der Projektoranordnung (110) verbunden ist, wobei das von der Lichtquelle projizierte Licht durch die Maske (140) projiziert wird,
**dadurch gekennzeichnet, dass** die Maske (140) ein Muster (142) zum Projizieren auf den gewünschten Abschnitt (D) des Operationssitus (S) umfasst, wobei das telezentrische Objektiv (135) derart zum Projizieren des Musters (142) ausgelegt ist, dass das Muster (142) beim Projizieren auf den gewünschten Abschnitt (D) des Operationssitus (S) nicht vergrößert wird.

2. System nach Anspruch 1, wobei das projizierte Licht mehrere Lichtwellenlängen zum Messen verschiedener Merkmale eines Gewebes innerhalb des Operationssitus (S) beinhaltet.

3. System nach Anspruch 1 oder Anspruch 2, wobei das Muster (142) konzentrische Ringe (142a) aufweist und jeder Ring (142a) einen Radius einer vorgegebenen Abmessung zum Projizieren der konzentrischen Ringe in den Operationssitus (S) repräsentiert.

4. System nach einem der vorhergehenden Ansprüche, wobei das Muster (142) eine Vielzahl von einheitlich beabstandeten Linien (142b) zum Projizieren der einheitlich beabstandeten Linien in den Operationssitus (S) aufweist.

5. System nach einem der vorhergehenden Ansprüche, wobei das Muster (142) eine einzige Linie (142c) zum Projizieren der einzigen Linie in den Operationssitus (S) aufweist.

6. System nach einem der vorhergehenden Ansprüche, wobei das Muster (142) einheitlich beabstandete Punkte (142d) zum Projizieren der einheitlich beabstandeten Punkte in den Operationssitus (S) aufweist.

7. System nach einem der vorhergehenden Ansprüche, wobei das Muster (142) eine Skala beinhaltet und die Skala auf den gewünschten Abschnitt (D) des Operationssitus (S) projiziert wird.

8. System nach einem der vorhergehenden Ansprüche, wobei die Lichtquelle mindestens einen Lichtstrahler (120) beinhaltet.

9. System nach einem der vorhergehenden Ansprüche, wobei die Lichtquelle weiterhin einen Diffusor zum Streuen des von dem mindestens einen Lichtstrahler (120) erzeugten Lichts beinhaltet.

10. System nach einem der vorhergehenden Ansprüche, wobei das telezentrische Objektiv (135) aus einem flexiblen Material ausgebildet ist, um das Einführen in den Operationssitus (S) zu unterstützen.

11. System nach einem der vorhergehenden Ansprüche, wobei die Maske (140) aus einem flexiblen Material ausgebildet ist, um das Einführen in den Operationssitus (S) zu unterstützen.

12. System nach einem der vorhergehenden Ansprüche, weiterhin aufweisend eine bildgebende Einheit (170), die zum Aufnehmen eines Bilds des projizierten Lichts im Operationssitus (S) ausgelegt ist.

13. System nach Anspruch 12, wobei die bildgebende Einheit (170) eine CMOS-Kamera ist.

14. System nach Anspruch 12 oder Anspruch 13, wobei die bildgebende Einheit (170) eine Rasterabtastvorrichtung ist.

15. System nach einem der Ansprüche 12 bis 14, ferner beinhaltend einen Mikroprozessor (175), der wirksam mit der bildgebenden Einheit (170) verbunden ist, wobei der Mikroprozessor (175) zum Durchführen von Parallaxenkorrekturen des aufgenommenen Bilds ausgelegt ist.

16. System nach Anspruch 15, weiterhin beinhaltend eine Anzeige (180), die wirksam mit dem Mikroprozessor (175) verbunden ist, wobei der Mikroprozessor (175) zum Berechnen von Bemaßungen des gewünschten Abschnitts (D) des Operationssitus (S) und zum Senden der Bemaßungen an die Anzeige (180) ausgelegt ist.

17. System nach einem der vorhergehenden Ansprüche, weiterhin aufweisend einen Sensor, der zum Durchführen von Triangulations- oder Abstandserfassungen ausgelegt ist.

## Revendications

1. Système de métrologie pour mesurer une partie souhaitée (D) d'un site chirurgical (S), comprenant :
une lentille télécentrique (135),
un ensemble de projecteur (110) comprenant une source de lumière pour projeter de la lumière à travers la lentille télécentrique (135) et dans le site chirurgical (S) ; et
un masque (140) couplé en service à l'ensemble de projecteur (110), dans lequel la lumière projetée par la source de lumière se projette à travers le masque (140),
**caractérisé en ce que** le masque (140) comprend un motif (142) pour projeter sur la partie souhaitée (D) du site chirurgical (S), la lentille télécentrique (135) étant configurée pour projeter le motif (142) de sorte que le motif (142) ne soit pas agrandi lorsqu'il est projeté sur la partie souhaitée (D) du site chirurgical (S).

2. Système selon la revendication 1, dans lequel la lumière projetée comprend de multiples longueurs d'onde de lumière pour la mesure de différentes caractéristiques du tissu dans le site chirurgical (S).

3. Système selon la revendication 1 ou la revendication 2, dans lequel le motif (142) présente des anneaux concentriques (142a) et chaque anneau (142a) représente un rayon d'une dimension donnée pour projeter les anneaux concentriques dans le site chirurgical (S).

4. Système selon l'une quelconque des revendications précédentes, dans lequel le motif (142) présente une pluralité de lignes uniformément espacées (142b) pour projeter les lignes uniformément espacées dans le site chirurgical (S).

5. Système selon l'une quelconque des revendications précédentes, dans lequel le motif (142) a une ligne unique (142c) pour projeter la ligne unique dans le site chirurgical (S).

6. Système selon l'une quelconque des revendications précédentes, dans lequel le motif (142) a des points uniformément espacés (142b) pour projeter les points uniformément espacés dans le site chirurgical (S).

7. Système selon l'une quelconque des revendications précédentes, dans lequel le motif (142) comprend une échelle et l'échelle est projetée sur la partie souhaitée (D) du site chirurgical (S).

8. Système selon l'une quelconque des revendications précédentes, dans lequel la source de lumière comprend au moins un émetteur de lumière (120).

9. Système selon l'une quelconque des revendications précédentes, dans lequel la source de lumière comprend en outre un diffuseur pour diffuser la lumière produite par le au moins un émetteur de lumière (120).

10. Système selon l'une quelconque des revendications précédentes, dans lequel la lentille télémétrique (135) est formée d'un matériau souple pour aider à l'insérer dans le site chirurgical (S).

11. Système selon l'une quelconque des revendications précédentes, dans lequel le masque (140) est formé d'un matériau souple pour aider à l'insérer dans le site chirurgical (S).

12. Système selon l'une quelconque des revendications précédentes, ayant en outre une unité d'imagerie (170) configurée pour capturer une image de la lumière projetée dans le site chirurgical (S).

13. Système selon la revendication 12, dans lequel l'unité d'imagerie (170) est une caméra CMOS.

14. Système selon la revendication 12 ou la revendication 13, dans lequel l'unité d'imagerie (170) est un dispositif de balayage de trame.

15. Système selon l'une quelconque des revendications 12 à 14, comprenant en outre un microprocesseur (175) couplé en service à l'unité d'imagerie (170), le microprocesseur (175) étant configuré pour effectuer des corrections de parallaxe de l'image capturée.

16. Système selon la revendication 15, comprenant en outre un affichage (180) couplé en service au microprocesseur (175), le microprocesseur (175) étant configuré pour calculer les dimensions de mesure de la partie souhaitée (D) du site chirurgical (S) et transmettre les dimensions de mesure à l'affichage (180).

17. Système selon l'une quelconque des revendications précédentes, ayant en outre un capteur configuré pour effectuer une triangulation ou une détection de distances.
